(19) Europäisches Patentamt
European Patent Office
Office européen des brevets

(11) **EP 2 730 563 B1**

(12) **EUROPÄISCHE PATENTSCHRIFT**

(45) Veröffentlichungstag und Bekanntmachung des Hinweises auf die Patenterteilung:
**11.01.2017 Patentblatt 2017/02**

(51) Int Cl.:
***C07D 229/00*** *(2006.01)*

(21) Anmeldenummer: **12192416.1**

(22) Anmeldetag: **13.11.2012**

(54) **Verfahren zur Herstellung von MDI-Dimer**

Process for the preparation of MDI dimer

Procédé de fabrication de dimère MDI

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**14.05.2014 Patentblatt 2014/20**

(60) Teilanmeldung:
**16197899.4**

(73) Patentinhaber: **EMS-PATENT AG**
**7013 Domat / Ems (CH)**

(72) Erfinder: **Kaplan, Andreas Dr.rer.nat.**
**7000 Chur (CH)**

(74) Vertreter: **Pfenning, Meinig & Partner mbB**
**Patent- und Rechtsanwälte**
**Theresienhöhe 11a**
**80339 München (DE)**

(56) Entgegenhaltungen:
**EP-A1- 0 572 994     DE-A1- 1 643 170**
**DE-A1-102004 038 784     JP-A- 9 328 474**
**JP-A- H0 912 556     US-A- 5 565 527**

EP 2 730 563 B1

**Beschreibung**

Die Erfindung betrifft ein Verfahren zur Herstellung von 4,4'-Methylen-bis(phenylisocyanat)-Dimer (MDI-Dimer), das sich dadurch auszeichnet, dass ein MDI-Dimer erhalten wird, das in hoher Reinheit vorliegt. Das MDI-Dimer das nach dem erfindungsgemäßen Verfahren hergestellt wird, zeichnet sich dadurch aus, dass es im Wesentlichen frei von MDI sowie von Harnstoffderivaten ist.

4,4'-Methylenbis(phenylisocyanat)-Dimere (MDI-Dimer) sind bekannt. Das 4,4'-MDI-Dimer besitzt die CAS-Nr. 17589-24-1.

Aus der JP 09-328 474 der Nippon Polyurethane ist bereits ein Verfahren zur Herstellung eines MDI-Dimers bekannt. Bei diesem Verfahren wird zuerst das MDI in Ethylacetat gelöst und mit einem Katalysator versetzt. Das MDI-Dimer wird dann nach 24 Stunden abfiltriert. Wie aus der JP 09-328 474 hervorgeht (siehe Example 3) besteht aber das Reaktionsprodukt nur zu 81 % aus dem MDI-Dimer. Das nach dem japanischen Dokument erhaltene MDI-Dimer weist somit noch hohe Anteile an MDI-Monomer bzw. Harnstoffderivate auf. Derartige MDI-Dimere sind deshalb auch als Vernetzer für Polymere nur bedingt geeignet.

Die EP 0 572 994 A1 offenbart ein Verfahren zur Herstellung von Uretdiongruppen aufweisenden Polyisocyanaten durch katalytische Dimerisierung von monomeren Isocyanaten.

Aus der US 5,565,527 sind polymere, katalytisch aktive Verbindungen mit polymeren Ketten, an die terminale oder laterale Imidazolgruppen gebunden sind, bekannt. Diese Verbindungen werden bei der Dimerisierung von Isocyanaten verwendet.

Die JP H09-012556 A beschreibt ein Verfahren zur Herstellung von dimerem MDI aus Polyisocyanat.

Aus der DE 10 2004 038 784 A1 ist ein Verfahren zur Herstellung monomerarmer Polyisocyanate durch destillative Entfernung von monomerem Isocyanat aus monomeres Isocyanat enthaltenden Polyisocyanaten, Polyisocyanat-prepolymeren und/oder Polyisocyanatderivaten bekannt.

Die DE 1 643 170 bezieht sich auf die Herstellung von Diiaocyanatdimeren in wäßrigem Medium, insbesondere auf die Dimerisierung von aromatischen Diisooyanaten in wäßriger Dispersion, wobei eine wäßrige Suspension oder ein Latex eines aromatischen Diisocyanatdimeren erhalten wird

Für viele Anwendungen, insbesondere für die Anwendung als Vernetzer für Polymere ist es aber wichtig, dass das 4,4-MDI-Dimer in hoher Reinheit vorliegt. Insbesondere für die Anwendung als Vernetzer für Polyurethane besteht die Erfordernis, ein 4,4-MDI-Dimer zur Verfügung zu stellen, das frei von MDI-Monomer und Harnstoffderivaten ist. Für derartige Anwendungen ist es auch wichtig, dass das MDI-Dimer gut lagerbar ist und in einer einheitlichen Korngröße zur Verfügung steht.

Ausgehend hiervon ist es die Aufgabe der vorliegenden Erfindung ein Verfahren zur Herstellung eines 4,4-MDI-Dimer zur Verfügung zu stellen, das zu einem Produkt mit hoher Reinheit führt. Angestrebt wird dabei, dass das 4,4-MDI-Dimer frei von MDI-Monomer und von Harnstoffderivaten ist. Die Erfindung umfasst weiterhin ein entsprechendes 4,4-MDI-Dimer sowie die Verwendung des 4,4-MDI-Dimer als Vernetzer für Polyurethane.

Die Erfindung wird im Bezug auf das Verfahren durch die Merkmale des Patentanspruches 1 gelöst. Die Unteransprüche zeigen vorteilhafte Weiterbildungen auf.

Das erfindungsgemäße Verfahren zeichnet sich dadurch aus, dass in einem ersten Verfahrensschritt a) ein Lösungsmittel, das bereits einen Katalysator enthält, bereit gestellt wird, wobei das Lösungsmittel derart ausgewählt ist, dass es als Lösungsmittel für 4,4'-Methylenbis(phenylisocyanat) geeignet ist. Bei diesem ersten Verfahrensschritt ist es zudem erforderlich, dass in einer Inertgasatmosphäre bei möglichst geringen Temperaturen, nämlich bei > 25°C bis max. 45°C gearbeitet wird.

Im nächsten Verfahrensschritt b) wird geschmolzenes und/oder gelöstes 4,4-Methylenbis(phenylisocyanat), d.h. MDI unter Rühren zugegeben. Im Folgenden beginnt dann das Produkt auszufallen.

Anschließend wird dann die Reaktion durch Zugabe eines Desaktivators abgebrochen (Verfahrensschritt c)). Bei diesem Verfahrensschritt ist es entscheidend, dass nach dem Abstoppen der Reaktion noch eine gewisse Zeitspanne weitergerührt wird. Diese Zeitspanne liegt im Bereich von 0,5 bis 1,5 Stunden.

In Anschluss an diesen Verfahrensschritt wird das MDI-Dimer vom Lösungsmittel getrennt (Verfahrensschritt d)) und im Verfahrensschritt e) gereinigt.

Beim erfindungsgemäßen Verfahren ist es dabei besonders wichtig, dass die genaue Abfolge der vorstehend beschriebenen Verfahrensschritte a) bis e) exakt eingehalten wird. Besondere Bedeutung kommt dabei der Reaktionsführung zu, insbesondere im Hinblick auf das Temperaturniveau. Es ist wesentlich, dass wie im Verfahrensschritt a) angegeben, eine Temperatur eingehalten wird, die im Bereich > 25°C bis 45°C liegt.

Bevorzugt ist es dabei, wenn die Temperatur im Verfahrensschritt a) auf 30 bis 40°C gehalten wird.

Für die Zugabe des geschmolzenen und/oder gelösten MDI ist ein Zutropfen bevorzugt.

Die Bereitstellung des den Katalysator enthaltenen Lösungsmittel erfolgt unter Inertgasatmosphäre wobei das Inertgas ausgewählt ist aus Stickstoff, Argon und/oder Helium. Bevorzugt ist dabei der Wassergehalt dieser Inertgase auf max. 0,01 Vol.-% , bevorzugt auf max. 0,005 Vol.-% eingestellt.

Geeignete Katalysatoren die in der Lösung nach Verfahrensschritt a) enthalten sind, sind ausgewählt aus tertiären Phosphinen, aminosubstituierten Phosphinen, Imidazolen, Guanidinen, in 3-oder4-Position substituierte Pyridinen, in 3- und 4-Position substituierte Pyridinen, cyclischen Amidinen, Antimonpentafluorid, Bortrifluorid oder Mischungen davon.

Bevorzugt werden als Katalysatoren tertiäre, aliphatische oder gemischt aliphatisch-aromatische Phosphine, Trialkylphosphine, Tris(N,N-dialkylamino)phosphine, Dialkylimidazole, 4-N,N-Dialkylaminopyridine, Pyridine, die in 3- und 4-Position durch N-Atome substituiert sind, welche über Kohlenstoffsegmente - bevorzugt zweigliedrige, gesättigte Kohlenstoffsegmente - verbunden sind oder Mischungen davon eingesetzt.

Besonders bevorzugt werden als Katalysatoren Tris(N,N-dialkylamino)phosphine, 1,2- Dialkylimidazole, 4-N,N-Dialkylaminopyridine oder Mischungen davon eingesetzt.

Ganz besonders bevorzugt werden als Katalysatoren eingesetzt Tris(N,N-dimethylamino)phosphin, Tris(N,N-diethylamino)phosphin, 4-N,N-Dimethylaminopyridin, 4-N,N-Diethylaminopyridin, 1,2-Dimethylimidazol, 1,2-Diethylimidazol oder Mischungen davon.

Phosphine werden auch als Phosphane oder Phosphorane bezeichnet.

Als Desaktivator für die Abstoppung der Reaktion (Verfahrensschritt c)) können alle an und für sich gängigen Desaktivatoren verwendet werden. Beispiele hierfür sind starke Säuren, Säurechloride, Säureanhydride oder Alkylierungsmittel.

Bevorzugt werden als Desaktivator verwendet Chloressigsäure, Trichloressigsäure, Trifluoressigsäure, Methansulfonsäure, Perfluorbutansulfonsäure, Phosphorsäure, Chlorameisensäure, Benzoylchlorid, Dimethylcarbamidsäurechlorid, Essigsäureanhydrid, Bernsteinsäureanhydrid, Dimethylsulfat, Methyljodid, Toloulsulfonsäuremethylester oder Mischungen davon.

Besonders bevorzugt wird als Desaktivator Benzoylchlorid verwendet.

Die Abtrennung des MDI-Dimers vom Lösungsmittel (Verfahrensschritt d)) erfolgt mit einem oder mit einer Kombination aus mehreren mechanischen Trennverfahren zur Fest-Flüssig-Trennung. Bevorzugt wird das mindestens eine mechanische Trennverfahren ausgewählt aus der Gruppe bestehend aus Sedimentation, Filtration, Zentrifugation und das Abscheiden mit Zyklonen.

Bei der Sedimentation kann die Trennung mittels Gravitation oder Zentrifugalkraft erfolgen.

Bei der Filtration kann die Trennung durch Gravitation, Zentrifugalkraft oder Druckdifferenzen erfolgen. Bei den Druckdifferenzen kann es sich um Unterdruck (Vakuum) oder Überdruck handeln.

Die Filtration kann mittels Nutschen, Scheibenfilter, Trommelfilter, Blattfilter, Plattenfilter, Bandfilter, Scherspaltfilter, Filterpressen, Membranfilterpressen, Bandpressen oder Kombinationen davon erfolgen.

Die Zentrifugation kann mittels Vollmantel-Schneckenzentrifugen, Tellerzentrifugen, Scheibenzentrifugen, Schälzentrifugen, Schubzentrifugen, Becherzentrifugen, Dekantierzentrifugen, Separatoren oder Kombinationen davon erfolgen.

Beim Abscheiden mit Zyklonen werden bevorzugt Hydrozyklone verwendet.

Anschließend an den Verfahrensschritt e) kann wie auch im Prinzip aus dem Stand der Technik schon bekannt, eine Trocknung des MDI-Dimer erfolgen, die bevorzugt bei 40°C bis 60°C im Vakuum oder Inertgasstrom für 12 bis 24 Stunden durchgeführt wird. Besonders bevorzugt erfolgt die Trocknung bei 40 °C bis 50 °C im Vakuum für 12 bis 20 Stunden.

Bevorzugt wird das MDI-Dimer nach dem Verfahrenschritt e) getrocknet.

Lösungsmittel die für das erfindungsgemäße Verfahren geeignet sind, sind Lösungsmittel, die in der Lage sind, das MDI-Monomer bei den angegebenen Reaktionstemperaturen, d.h. bei > 25°C bis 45°C vollständig zu lösen. Der Wassergehalt des Lösungsmittels darf maximal 0,04 Gew.-%, bevorzugt maximal 0,02 Gew.-%, besonders bevorzugt maximal 0,01 Gew.-% betragen.

Beispiele für Lösungsmittel sind Aceton, N-Methylpyrrolidon, Benzol, Ethylacetat, Acetonitril, Nitromethan, Kerosin, Octan oder Mischungen hiervon.

Die Erfinder konnten dabei zeigen, dass als Lösungsmittel Ethylacetat besonders geeignet ist. Beim erfindungsgemäßen Verfahren wird bevorzugt bei der Reinigung des MDI-Dimers mit dem gleichen Lösungsmittel gearbeitet, dass auch für die Reaktion verwendet wird. Bevorzugt wird dabei somit sowohl für das MDI-Monomer wie auch für den Reinigungsschritt Ethylacetat verwendet.

Beschrieben wird weiterhin ein 4,4'-Methylenbis(phenylisocyanat)-Dimer, das nach einem Verfahren wie vorstehend beschrieben herstellbar ist. Das MDI-Dimer zeichnet sich dadurch aus, dass es im Wesentlichen frei von monomerem MDI ist. Unter im Wesentlichen frei von monomerem MDI wird verstanden, dass das MDI-Dimer weniger als 1 Gew.-%, bevorzugt weniger als 0,5 Gew.-%, besonders bevorzugt weniger als 0,1 Gew.-%, ganz besonders bevorzugt weniger als 0,08 Gew.-% monomeres MDI enthält. Das MDI-Dimer weist zudem keine mit HPLC-MS (High Performance Liquid Chromatography-Massenspektrometrie-Kombination) mit UV-Detektor und Massendetektor nachweisbaren Anteile an Produkten wie Trimere, Tetramere, Pentamere oder noch höhere Oligomere auf. Das MDI-Dimer ist auch im Wesentlichen frei von Harnstoffderivaten. Unter im Wesentlichen frei von Harnstoffderivaten wird verstanden, dass das MDI-Dimer weniger als 10 Gew.-%, bevorzugt weniger als 7 Gew.-%, besonders bevorzugt weniger als 4 Gew.-%, ganz besonders bevorzugt weniger als 2 Gew.-% Harnstoffderivate enthält.

Das hochreine MDI-Dimer wird bevorzugt in einer Pulverform zur Verfügung gestellt. Dazu kann das MDI-Dimer nach dem Verfahrensschritt d) (Abtrennen des erhaltenen MDI-Dimer vom Lösungsmittel) und der Reinigung des erhaltenen MDI-Dimer mit den Lösungsmittel (Verfahrensschritt e)) noch einem Mahlprozess unterzogen werden, sodass dann eine mittlere Granulatgröße $d_{50}$ der Pulver vorliegt, die im Bereich zwischen 1 und 4 $\mu$m, bevorzugt bei 2 $\mu$m liegt.

Das hochreine MDI-Dimer ist besonders geeignet als Vernetzer für Polymere und hier insbesondere für Polyurethane zur Herstellung von Schaum- und Dämmstoffen, z.B. als Weich-, Hart- oder Integralschaum, oder von ungeschäumten festen oder gummielastischen Werkstoffen für technische Artikel, wie z.B. Sitzmöbel, Gießharze, Lackharze, Dichtstoffe, Klebeharze oder Beschichtungsmassen für Kunstleder und Textilhilfsmittel. Es hat sich gezeigt, dass das MDI-Dimer eine äußerst große Reaktionsfähigkeit aufweist und dann zu Endprodukten führt, die nach der Vernetzung exzellente mechanische Eigenschaften aufweisen.

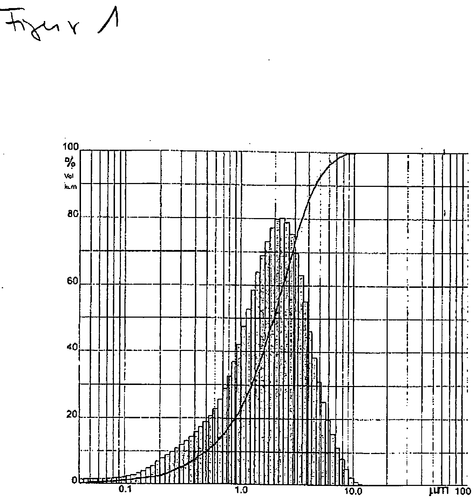

Die Erfindung wird nachfolgend anhand eines allgemeinen Ausführungsbeispiels und eines speziellen Herstellungsbeispiels sowie anhand der Figur 1 näher erläutert.

Figur 1 zeigt die Partikelgrößenverteilung eines erfindungsgemäß hergestellten MDI-Dimer, dass nach dem Verfahrensschritt e) noch einer Mahlung unterzogen worden ist. Wie aus Figur 1 hervorgeht, weist das MDI-Dimer in diesem Falle eine sehr enge Größenverteilung auf. Ein MDI-Dimer in einer wie vorstehend beschriebenen hochreinen Form in Kombination mit der sehr feinen Partikelverteilung eignet sich besonders gut als Vernetzer für Polyurethane. Es wurde dabei eine sehr hohe Reaktivität festgestellt, die einerseits auf die hohe Reinheit und andererseits auf die Partikelgrößenverteilung zurückgeführt wird. Weiterhin ist ein MDI-Dimer wie vorstehend beschrieben ausgezeichnet lagerfähig und deshalb besonders gut geeignet als Vernetzer für Polyurethane.

Die Herstellung des MDI-Dimers erfolgt in Lösung in einer inerten Atmosphäre unter Wasserausschluss bei 30 bis 40 °C. Das Lösungsmittel enthaltend den gelösten Katalysator wird vorgelegt. Dazu wird unter Rühren geschmolzenes oder gelöstes MDI innerhalb von 0,5 bis 3 h zugetropft. Nach etwa der Hälfte der Zugabe beginnt das Produkt auszufallen. Nach dem Zutropfen wird noch 1 bis 4 h bei der Reaktiontemperatur gerührt. Anschliessend wird die Reaktion durch Zugabe des Desaktivators abgebrochen, wobei nach der Zugabe noch weitere 0,5 bis 1,5 h bei der Reaktionstemperatur gerührt wird. Der Ansatz wird nach dem Abkühlen auf Raumtemperatur durch eine Glas- oder Keramikfritte im Vakuum filtriert und anschliessend mindestens zweimal mit Lösungsmittel gewaschen. Die Trocknung findet im Vakuum oder Inertgasstrom bei 40 bis 60 °C für 12 bis 24 h statt.

Das so hergestellte MDI-Dimer enthält weder Trimere, Tetramere, Pentamere noch höhere Oligomere, welche mittels HPLC-MS mit UV-Detektor und Massendetektor nachweisbar sind.

Als Inertgase werden Stickstoff, Argon oder Helium verwendet, deren Wassergehalt maximal 0,01 Vol.-% beträgt.

Das Lösungsmittel wird auf einen Wassergehalt von maximal 0,04 Gew.-% getrocknet.

Sollte für die Reaktion geschmolzenes MDI verwendet werden, wird es bei maximal 60 °C, bevorzugt bei 45 bis 55 °C aufgeschmolzen.

**Bestimmung des Gehalts an monomerem MDI im MDI-Dimer**

Das monomere MDI wird aus dem MDI-Dimer extrahiert und im Extrakt mit Ethanol derivatisiert.

Das MDI-Dimer wird dazu mit 25 ml Ethylacetat in einem 50 ml Rundkolben fünf Stunden bei 23 °C extrahiert, wobei mit einem Magnetrührer gerührt wird. Der Extrakt wird filtriert und das Filtrat in einen 50 ml-Messkolben überführt. Anschliessend wird der Extrakt mit Ethanol auf 50 ml aufgefüllt, bei 23 °C 30 min stehen gelassen, filtriert und in ein Glasfläschchen abgefüllt.

Dieser Vorgang wird mit einer niedrigen Einwaage (1 mg MDI-Dimer pro ml Ethylacetat) und mit einer hohen Einwaage (25 mg MDI-Dimer pro ml Ethylacetat) durchgeführt.

Die Bestimmung der Konzentration an monomerem MDI im Extrakt erfolgt mit HPLC (High Performance Liquid Chromatography) nach der Methode des externen Standards.

Als HPLC Gerät wird dabei ein Acquity UPLC der Firma Waters Corp. verwendet, als Säule BEH C18 von Waters Corp. (10,0 cm lang, Innendurchmesser 2,1 mm, Partikelgrösse der Füllung 1,7 $\mu$m). Als Eluent dient eine Acetonitril/Wasser-Gradienten-Mischung mit einer Flussrate von 0,4 ml/min. Die Detektion erfolgt mit einem UV-Detektor bei einer Wellenlänge von 254 nm.

Als externer Standard wird MDI verwendet. Mindestens 3 Einwaagen werden eingewogen, in 50mL Ethylacetat/Ethanol 1/1 (vol/vol) gelöst und abgefüllt. Die Kalibriergerade wird mit der Software Empower 2 von Waters Corp. erstellt und muss einen Korrelationskoeffizienten von mindestens 0.999 aufweisen.

Der Gehalt des monomeren MDI in Gew.-% wird nach der folgenden Formel berechnet:

$$\text{monomeres MDI} \; = \; ([(KA - KB) \times V] : EA) \times 100$$

KA    Konzentration des monomeren MDI in mg/ml bei hoher Einwaage
KB    Konzentration des monomeren MDI in mg/ml bei niedriger Einwaage
V     Volumen Ethylacetat in ml
EA    Einwaage des MDI-Dimeren bei hoher Einwaage in mg

Acetonitril, Ethanol und Eythylacetat werden in der Reinheit p.a. (zur Analyse) verwendet. Bei Wasser handelt es sich um bidestilliertes Wasser.

Angegeben wird der arithmetische Mittelwert aus drei Messungen.

**Bestimmung der Reinheit des MDI-Dimeren**

Die Reinheit des MDI-Dimeren wird mit HPLC-MS (High Performance Liquid Chromatography-Massenspektrometrie-Kombination) mit UV-Detektor und Massendetektor bestimmt, wobei die Zuordnung der Peaks über die Masse erfolgt. Die Reinheit des MDI-Dimeren wird in Flächen-% (Fl.-%) angegeben, die Gesamtfläche aller Peaks entspricht dabei 100 Fl.-%. Die angegebenen Werte sind der arithmetische Mittelwert aus drei Messungen.

10 mg des MDI-Dimeren werden dazu in einer Mischung aus 5 ml Tetrahydrofuran und 5 ml Methanol bei 23 °C gelöst, zur Derivatisierung mit dem Methanol danach noch 30 min stehen gelassen, filtriert und in ein Glasfläschchen abgefüllt.

Als HPLC Gerät wird ein Acquity UPLC der Firma Waters Corp. verwendet, als Säule BEH C18 der Waters Corp. (10,0 cm lang, Innendurchmesser 2,1 mm, Partikelgrösse der Füllung 1,7 $\mu$m). Als Eluent dient eine Acetonitril/Wasser-Gradienten-Mischung. Der UV-Detektor arbeitet bei 254 nm und der Massendetektor SQ im ESI-Modus (Elektro-Spray-Ionisations-Modus).

Acetonitril, Methanol und Tetrahydrofuran werden in der Reinheit p.a. (zur Analyse) verwendet. Bei Wasser handelt es sich um bidestilliertes Wasser.

**Herstellungsbeispiele für 4,4'-Methylenbis(phenylisocyanat)-Dimer**

Die Reaktionsapparatur besteht aus einem doppelwandigen, zylindrischem Reaktionsgefäss (Volumen 1 l) mit Bodenablassventil, Rührer, Schliffdeckel, drei Tropftrichtern, zwei davon doppelwandig, einer mit dem Bodenablassventil verbundenen G4-Fritte und einer Vakuumpumpe.

Die Apparatur wird dreimal im Wechsel von Vakuum (30 mbar) und Stickstoff inertisiert. Die gesamte Herstellung des MDI-Dimers findet unter einem Stickstoffstrom statt.

Im auf 35 °C thermostatisierten Reaktiongefäss werden mittels eines Tropftrichters 500 ml Ethylacetat in dem 1,0 g 4-N,N-Dimethylaminopyridin gelöst ist vorgelegt und im Reaktionsgefäss auf 35 °C thermostatisiert.

130,0 g im Umluftofen bei 50 °C aufgeschmolzenes MDI werden über einen auf 50 °C thermostatisierten Tropftrichter in 45 Minuten unter Rühren zugegeben. Dabei wird darauf geachtet, dass die Temperatur des Reaktionsgemisches bei 35 ° C bleibt. Nach etwa der Hälfte der Zugabe beginnt das Produkt auszufallen.

Im Anschluss an die MDI-Zugabe wird noch 2 Stunden bei 35 °C gerührt.

Danach werden 1,27 g Benzoylchlorid gelöst in 5 ml Ethylacetat über einen auf 35 °C thermostatisierten Tropftrichter in 10 Minuten unter Rühren zugegeben und weitere 15 min gerührt.

Anschliessend wird der Ansatz auf 23 °C abgekühlt und durch die G4-Fritte bei 550 mbar filtriert. Das Produkt wird zweimal mit je 100 ml Ethylacetat gewaschen und bei 50 °C und 30 mbar 16 h getrocknet.

Ausbeute: 120,5 g, d.h. 92,7 Gew.-% bezogen auf die MDI-Einwaage.

Das Produkt enthält:

| | | |
|---|---|---|
| 0,07 | Gew.-% | monomeres MDI* |
| 99,5 | Fl.-% | MDI-Dimer, derivatisiert** |
| 0,5 | Fl.-% | Harnstoff-Dimer, derivatisiert** |

\* bestimmt durch Extraktion und anschliessender HPLC

\*\* bestimmt durch HPLC-MS, angegeben in Flächen-% (Fl.-%)

**Patentansprüche**

1. Verfahren zur Herstellung von 4,4'-Methylenbis(phenylisocyanat)-Dimer (MDI-Dimer) mit folgenden Schritten:

   a) Bereitstellen eines einen Katalysator enthaltenen Lösungsmittel für 4,4'-Methylenbis(phenylsiocyanat) (MDI) mit einer Inertgasatmosphäre bei > 25°C bis 45°C,
   b) Zugabe eines geschmolzenen und/oder gelösten MDI unter Rühren über eine Zeitspanne von 0,5 bis 3 Stunden,
   c) Zugabe eines Desaktivators, mit der Maßgabe, dass noch über eine Zeitspanne von 0,5 bis 1,5 Stunden bei der gegebenen Reaktionstemperatur gerührt wird,
   d) Abtrennen des erhaltenen MDI-Dimer vom Lösungsmittel und
   e) Reinigung des erhaltenen MDI-Dimer mit dem Lösungsmittel.

2. Verfahren nach Anspruch 1, **dadurch gekennzeichnet, dass** in Verfahrensschritt a) eine Temperatur im Bereich von 30°C bis 40°C eingehalten und das geschmolzene und/oder gelöste MDI (Verfahrensschritt b) zugetropft wird.

3. Verfahren nach Anspruch 1 oder 2, **dadurch gekennzeichnet, dass** das Inertgas ausgewählt ist aus Stickstoff, Argon und/oder Helium, deren Wassergehalt max. 0,01 Vol.-%, insbesondere 0,005 Vol.-% beträgt.

4. Verfahren nach mind. einem der Ansprüche 1 bis 3, **dadurch gekennzeichnet, dass** der Katalysator ausgewählt ist aus der Gruppe bestehend aus tertiären Phosphinen, aminosubstituierten Phosphinen, Imidazolen, Guanidinen, in 3- oder 4-Position substituierten Pyridinen, in 3- und 4-Position substituierten Pyridinen, cyclischen Amidinen, Antimonpentafluorid, Bortrifluorid und Mischungen davon, bevorzugt ausgewählt aus der Gruppe bestehend aus tertiären, aliphatischen oder gemischt aliphatisch-aromatischen Phosphinen, Trialkylphosphinen, Tris(N,N-dialkylamino)phosphinen, Dialkylimidazolen, 4-N,N-Dialkylaminopyridinen, Pyridinen, die in 3- und 4-Position durch N-Atome substituiert sind, welche über Kohlenstoffsegmente, insbesondere zweigliedrige, gesättigte Kohlenstoffsegmente, verbunden sind und Mischungen davon, besonders bevorzugt ausgewählt aus der Gruppe bestehend aus Tris(N,N-dialkylamino)phosphinen, 1,2- Dialkylimidazolen, 4-N,N-Dialkylaminopyridinen und Mischungen davon und ganz besonders bevorzugt ausgewählt aus der Gruppe bestehend aus Tris(N,N-dimethylamino)phosphin, Tris(N,N-diethylamino)phosphin, 4-N,N-Dimethylaminopyridin, 4-N,N-Diethylaminopyridin, 1,2-Dimethylimidazol, 1,2-Diethylimidazol und Mischungen davon.

5. Verfahren nach mind. einem der Ansprüche 1 bis 4, **dadurch gekennzeichnet, dass** der Desaktivator ausgewählt ist aus der Gruppe bestehend aus starken Säuren, Säurechloriden, Säureanhydriden oder Alkylierungsmitteln, bevorzugt ausgewählt aus der Gruppe bestehend aus Chloressigsäure, Trichloressigsäure, Trifluoressigsäure, Methansulfonsäure, Perfluorbutansulfonsäure, Phosphorsäure, Chlorameisensäure, Benzoylchlorid, Dimethylcarbamidsäurechlorid, Essigsäureanhydrid, Bernsteinsäureanhydrid, Dimethylsulfat, Methyljodid oder Toloulsulfonsäuremethylester und besonders bevorzugt Benzoylchlorid.

6. Verfahren nach Anspruch 1 bis 5, **dadurch gekennzeichnet, dass** die Abtrennung des Reaktionsproduktes in Verfahrensschritt d) mittels mit einem oder mit einer Kombination aus mehreren mechanischen Trennverfahren zur Fest-Flüssig-Trennung erfolgt, wobei die mechanischen Trennverfahren bevorzugt ausgewählt sind aus der Gruppe bestehend aus Sedimentation, Filtration, Zentrifugation und dem Abscheiden mit Zyklonen.

7. Verfahren nach mind. einem der Ansprüche 1 bis 6, **dadurch gekennzeichnet, dass** nach dem Verfahrensschritt e) eine Trocknung, bevorzugt im Vakuum bei 40°C bis 60°C für 12 bis 24 Stunden durchgeführt wird.

8. Verfahren nach mind. einen der Ansprüche 1 bis 7, **dadurch gekennzeichnet, dass** das Lösungsmittel ausgewählt ist aus der Gruppe bestehend aus Aceton, N-Methylpyrrolidon, Benzol, Ethylacetat, Acetonitril, Nitromethan, Kerosin, Octan und Mischungen hiervon.

9. Verfahren nach Anspruch 8, **dadurch gekennzeichnet, dass** das Lösungsmittel Ethylacetat ist, dessen Wassergehalt bevorzugt max. 0,04 Gew.-%, besonders bevorzugt maximal 0,02 Gew.-% und ganz besonders bevorzugt 0,01 Gew.-%beträgt.

**Claims**

1. Method for the production of 4,4'-methylenebis(phenylisocyanate) dimer (MDI dimer) having the following steps:

   a) provision of a solvent comprising a catalyst for 4,4'-methylenebis(phenylisocyanate) (MDI) with an inert gas atmosphere at > 25°C to 45°C,
   b) addition of a melted and/or dissolved MDI with stirring over a timespan of 0.5 to 3 hours,
   c) addition of a deactivator, with the proviso that stirring takes place in addition over a timespan of 0.5 to 1.5 hours at the given reaction temperature,
   d) separation of the obtained MDI dimer from the solvent and
   e) purification of the obtained MDI dimer with the solvent.

2. Method according to claim 1, **characterised in that**, in method step a), a temperature in the range of 30°C to 40°C is maintained and the melted and/or dissolved MDI (method step b) is added in drops.

3. Method according to claim 1 or 2, **characterised in that** the inert gas is selected from nitrogen, argon and/or helium, the water content of which is max. 0.01% by volume, in particular 0.005% by volume.

4. Method according to at least one of the claims 1 to 3, **characterised in that** the catalyst is selected from the group consisting of tertiary phosphines, amino-substituted phosphines, imidazoles, guanidines, pyridines substituted in position 3 or 4, pyridines substituted in position 3 and 4, cyclic amidines, antimony pentafluoride, boron trifluoride and mixtures thereof, preferably selected from the group consisting of tertiary, aliphatic or mixed aliphatic-aromatic phosphines, trialkylphosphines tris(N,N-dialkylamino)phosphines, dialkylimidazoles, 4-N,N-dialkylaminopyridines, pyridines which are substituted by N atoms in position 3 and 4 and which are connected via carbon segments, in particular binary, saturated carbon segments, and mixtures thereof, selected for particular preference from the group consisting of tris(N,N-dialkylamino)phosphines, 1,2-dialkylimidazoles, 4-N,N-dialkylaminopyridines and mixtures thereof and selected for very particular preference from the group consisting of tris(N,N-dimethylamino)phosphine, tris(N,N-diethylamino)phosphine, 4-N,N-dimethylaminopyridine, 4-N,N-diethylaminopyridine, 1,2-dimethylimidazole, 1,2-diethylimidazole and mixtures thereof.

5. Method according to at least one of the claims 1 to 4, **characterised in that** the deactivator is selected from the group consisting of strong acids, acid chlorides, acid anhydrides or alkylation agents, preferably selected from the group consisting of chloroacetic acid, trichloroacetic acid, trifluoroacetic acid, methane sulphonic acid, perfluorobutane sulphonic acid, phosphoric acid, chloroformic acid, benzoyl chloride, dimethyl carbamide acid chloride, acetic acid anhydride, succinic acid anhydride, dimethyl sulphate, methyl iodide, toluene sulphonic acid methyl ester and mixtures thereof and, for particular preference, benzoyl chloride.

6. Method according to claim 1 to 5, **characterised in that** the separation of the reaction product in method step d) is effected by means of one or by means of a combination of a plurality of mechanical separation methods for solid-liquid separation, the mechanical separation methods being selected preferably from the group consisting of sedimentation, filtration, centrifugation and deposition with cyclones.

7. Method according to at least one of the claims 1 to 6, **characterised in that**, after method step e), a drying, preferably in a vacuum or inert gas flow at 40°C to 60°C, is implemented for 12 to 24 hours.

8. Method according to at least one of the claims 1 to 7, **characterised in that** the solvent is selected from the group consisting of acetone, N-methylpyrrolidone, benzene, ethylacetate, acetonitrile, nitromethane, kerosine, octane and mixtures hereof, particularly preferred ethylacetate.

9. Method according to at least one of the claims 1 to 8, **characterised in that** the water content of the solvent is preferably max. 0.04% by weight, particularly preferred at most 0.02% by weight and very particularly preferred 0.01% by weight.

**Revendications**

1. Procédé de préparation de bis(phénylisocyanato)-4,4'-méthylène dimère (MDI-dimère), comportant les étapes suivantes :

a) fourniture d'un solvant contenant un catalyseur pour le bis(phénylisocyanato)-4,4'-méthylène (MDI) sous atmosphère d'un gaz inerte à une température de > 25°C à 45°C,

b) addition d'un MDI fondu et/ou dissous, sous agitation, sur un laps de temps de 0,5 à 3 heures,

c) addition d'un désactivateur, à la condition que l'on agite encore pendant un laps de temps de 0,5 à 1,5 heures à la température de réaction indiquée,

d) séparation, du solvant, du MDI-dimère obtenu, et

e) purification du MDI-dimère obtenu, avec le solvant.

2. Procédé selon la revendication 1, **caractérisé en ce que**, dans l'étape a) du procédé, on maintient une température comprise dans la plage de 30°C à 40°C, et on ajoute goutte à goutte le MDI fondu et/ou dissous (étape b) du procédé).

3. Procédé selon la revendication 1 ou 2, **caractérisé en ce que** le gaz inerte est choisi parmi l'azote, l'argon et/ou l'hélium, dont la teneur en eau est au maximum de 0,01 % en volume, en particulier de 0,005 % en volume.

4. Procédé selon au moins l'une des revendications 1 à 3, **caractérisé en ce que** le catalyseur est choisi dans le groupe consistant en les phosphines tertiaires, les phosphines amino-substituées, les imidazoles, les guanidines, les pyridines substituées en position 3 ou 4, les pyridines substituées en positions 3 et 4, les amidines cycliques, le pentafluorure d'antimoine, le trifluorure de bore et les mélanges de ceux-ci, de préférence choisi dans le groupe consistant en les phosphines tertiaires, aliphatiques, ou aliphatiques aromatiques en mélange, les trialkylphosphines, les tris(N,N-dialkylamino)phosphines, les dialkylimidazoles, les 4-N,N-dialkylaminopyridines, les pyridines qui sont substituées en positions 3 et 4 par des atomes d'azote qui sont reliés par des segments carbonés, en particulier des segments carbonés saturés à deux chaînons, et les mélanges de ceux-ci, d'une manière particulièrement préférée choisi dans le groupe consistant en les tris(N,N-dialkylamino)phosphines, les 1,2-dialkylimidazoles, les 4-N,N-dialkylminopyridines et les mélanges de ceux-ci, et d'une manière tout particulièrement préférée choisi dans le groupe consistant en la tris(N,N-diméthylamino)phosphine, la tris(N,N-diéthylamino)phosphine, la 4-N,N-diméthylaminopyridine, la 4-N,N-diéthylaminopyridine, le 1,2-diméthylimidazole, le 1,2-diéthylimidazole et les mélanges de ceux-ci.

5. Procédé selon au moins l'une des revendications 1 à 4, **caractérisé en ce que** le désactivateur est choisi dans le groupe consistant en les acides forts, les chlorures d'acyle, les anhydrides d'acide ou les agents d'alkylation, de préférence choisi dans le groupe consistant en l'acide chloracétique, l'acide trichloracétique, l'acide trifluoracétique, l'acide méthanesulfonique, l'acide perfluorobutanesulfonique, l'acide phosphorique, l'acide chloroformique, le chlorure de benzoyle, le chlorure de l'acide diméthylcarbamique, l'anhydride acétique, l'anhydride succinique, le sulfate de diméthyle, l'iodure de méthyle ou l'ester méthylique de l'acide toluènesulfonique, et d'une manière particulièrement préférée le chlorure de benzoyle.

6. Procédé selon les revendications 1 à 5, **caractérisé en ce que** la séparation du produit de la réaction dans l'étape d) du procédé a lieu à l'aide d'un procédé de séparation mécanique conduisant à une séparation solide-liquide, ou d'une combinaison de plusieurs de ceux-ci, les procédés de séparation mécanique étant de préférence choisis dans le groupe consistant en la sédimentation, la filtration, la centrifugation et la séparation avec des cyclones.

7. Procédé selon au moins l'une des revendications 1 à 6, **caractérisé en ce qu'**on procède après l'étape e) du procédé à un séchage, de préférence sous vide à 40°C à 60°C pendant 12 à 24 heures.

8. Procédé selon au moins l'une des revendications 1 à 7, **caractérisé en ce que** le solvant est choisi dans le groupe consistant en l'acétone, la N-méthylpyrrolidone, le benzène, l'acétate d'éthyle, l'acétonitrile, le nitrométhane, le kérosène, l'octane et les mélanges de ceux-ci.

9. Procédé selon la revendication 8, **caractérisé en ce que** le solvant est l'acétate d'éthyle, dont la teneur en eau est de préférence au maximum de 0,04 % en poids, d'une manière particulièrement préférée au maximum de 0,02 % en poids et d'une manière tout particulièrement préférée de 0,01 % en poids.

Figur 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 9328474 A
- EP 0572994 A1
- US 5565527 A
- JP H09012556 A
- DE 102004038784 A1
- DE 1643170